# EUROPÄISCHE PATENTSCHRIFT

(11) **EP 0 553 773 B1**
(45) Veröffentlichungstag und Bekanntmachung des Hinweises auf die Patenterteilung: **08.04.1998**
(21) Anmeldenummer: 93101149.8
(22) Anmeldetag: 26.01.1993
(51) Int. Cl.: G01N 33/543, G01N 33/558, G01N 33/78

(54) **Analyseelement für Immunoassays**
Analytical element for immunoassays
Elément analytique pour les essais immunologiques

(30) Priorität: 31.01.1992 DE 4202848
(43) Veröffentlichungstag der Anmeldung: 04.08.1993
(73) Patentinhaber: BOEHRINGER MANNHEIM GMBH, 68298 Mannheim (DE)
(72) Erfinder: Finke, Werner, Dr., W-6141 Einhausen (DE); Gaa, Otto, Dr., W-6520 Worms 21 (DE); Goerlach-Graw, Ada, Dr., W-6714 Weisenheim am Sand (DE); Nägele, Ulrich, Dr., W-6940 Weinheim (DE); Unkrig, Volker, Dr., W-6802 Ladenburg (DE); Wilk, Hans-Erich, Dr., W-6141 Einhausen (DE)

(56) Entgegenhaltungen:
- EP-A- 0 120 602
- EP-A- 0 344 578
- EP-A- 0 469 444
- EP-A- 0 469 445
- WO-A-82/02601
- DE-C- 3 445 816
- FR-A- 2 355 290

## Beschreibung

Die Erfindung betrifft ein Analysenelement zur Bestimmung eines Analyten in einer Probenflüssigkeit nach dem Prinzip eines heterogenen Immunoassays aus einem porösen kapillaraktiven Trägermaterial mit einer Multifunktionszone, auf der ein trägerfixierter spezifischer Bindungspartner immobilisiert ist, und einer die Multifunktionszone umgebenden Chromatographiezone. Weiterhin betrifft die Erfindung ein Verfahren zur Bestimmung eines Analyten in einer Probenflüssigkeit nach dem Prinzip eines heterogenen Immunoassays unter Verwendung dieses Analysenelementes.

Auf dem Gebiet der medizinischen Diagnostik ist die Zahl der in physiologischen Probenflüssigkeiten zu bestimmenden Substanzen explosionsartig angewachsen. Zur Bestimmung dieser Substanzen oder Analyten kommen immunologischen Nachweisverfahren eine zunehmende Bedeutung zu. Hervorzuheben sind dabei heterogene Immunoassays, bei denen normalerweise ein analytspezifischer bioaffiner Bindungspartner an einer Festphase gebunden vorliegt. Allgemein wird dabei zwischen kompetitiven Immunoassays und Sandwich-Immunoassays unterschieden.

Bei kompetitiven Immunoassays konkurriert eine vorbestimmte Menge eines markierten Analytderivates mit den zu bestimmenden Analytmolekülen um die Bindungsstelle des festphasengebundenen Bindungspartners. Nach einer Inkubationsphase wird das nichtgebundene Material ausgewaschen und die Menge des markierten Analytderivates in der gebundenen oder in der freien Phase als Maß für die Menge des Analyten bestimmt.

Bei Sandwich-Immunoassays wird im allgmeinen nach der Bindung des Analyten an den festphasengebundenen analytspezifischen Bindungspartner ein zweiter analytspezifischer Bindungspartner, der eine meßbare Markierung trägt, im Überschuß zugegeben. Nach Auswaschen des nichtgebundenen Materials wird die Menge des markierten Reagenzes in der gebundenen Phase als Maß für die Analytmenge bestimmt.

Seit einiger Zeit wurden heterogene Immunoassays auf sogenannten Testträgern aus porösen oder faserigen Materialien vorgeschlagen. Die chromatographische Eigenschaft dieser Testträger dient dazu, gebundenes von nichtgebundenem markiertem Reagenz zu trennen.

EP-A-0 070 300 beschreibt ein Testelement aus einer porösem Membran, die in zwei Zonen aufgeteilt ist. Auf einer zentralen Reaktionszone ist ein analytspezifischer Bindungspartner immobilisiert. Die Reaktionszone ist radial von einer Chromatographiezone umgeben, die durch Kapillarkräfte Flüssigkeit aus der Reaktionszone ziehen kann. Je nach Testführung wird der Analyt auf die Reaktionszone aufgegeben und gleichzeitig oder anschließend eine vorbestimmte Menge eines markierten Bindungspartners zugegeben. Durch Aufgabe von Waschflüssigkeit wird ungebundenes Markierungsreagenz aus der Reaktionszone in die Chromatographiezone entfernt und das gebundene Markierungsreagenz in der Reaktionszone gemessen.

Nachteilig für die Testführung, für die Testdauer und die Genauigkeit wirkt sich aus, daß insbesondere bei kompetitiver Testführung das markierte Bindungsreagenz in dosierter Form separat zugegeben werden muß, um eine vorzeitige Wechselwirkung von immobilisiertem und markiertem Bindungsreagenz vor der Analytzugabe in der Reaktionszone zu verhindern. Die langen Testzeiten und die komplizierte Handhabung solcher Testträger sind mit dem Einsatz in der Notfalldiagnose nicht zu vereinbaren.

Es wurden verschiedene Vorschläge gemacht, die Zahl der Reagenzdosierschritte zu reduzieren und alle für die heterogene Nachweisreaktion notwendigen Bindungspartner auf einem Testträger zu integrieren.

US-Patent 4,361,537, EP-A-0 291 194 oder EP-A-0 186 799 beschreiben beispielsweise chromatographische Teststreifen, bei denen die Analytaufgabezone und die Reaktionszone, die den immobilisierten Bindungspartner enthält, auf der Ebene des Teststreifens voneinander getrennt sind. Zwischen diesen beiden Zonen befinden sich eine oder mehrere separate Zonen, auf denen Immunreagenzien ablösbar imprägniert sind. Bei der chromatographischen Wanderung des Analyten von der Aufgabe-zone zur Reaktionszone werden die Immunreagenzien nacheinander gelöst und sollten idealerweise zusammen mit dem Analyten in die Reaktionszone gelangen. Dieses ist jedoch häufig nicht der Fall, bedingt durch unterschiedliche chromatographische Wanderungsgeschwindigkeiten von Analyt und markiertem Bindungsreagenz. Ferner gehen die Reagenzien auf ihrer chromatographischen Wanderung oft unspezifische Bindungen mit den Poren des Trägermaterials ein. Die Zusammensetzung der chromatographiefront verändert sich ständig und somit auch die Bindungsgleichgewichte der Immunbindungspartner. So können diese Teststreifen häufig nur für qualitative oder halbquantitative Analysen eingesetzt werden.

In US-Patent 4,446,232 und EP-A-0 279 097 werden Testträger beschrieben, bei denen die verschiedenen miteinander unverträglichen Immunreagenzien in übereinandergeschichteten Funktionszonen imprägniert sind. Der Flüssigkeitsstrom muß daher senkrecht und nicht mehr parallel zur Schichtenebene verlaufen. Nach Aufgabe der Probe auf die oberste Schicht fungiert die unterste Schicht als Detektionszone, in der die nichtgebundenen markierten Bindungspartner gemessen werden. Bei der geringen Dicke der Schichten ist die Verweildauer der Probenflüssigkeit innerhalb der einzelnen Schichten sehr kurz im Vergleich zu der für heterogene Reaktionen benötigten Reaktionszeit. Weiterhin kann es innerhalb der Schichten zur Rückdiffussion von Reagenzien kommen. Da nur die freie Phase in der untersten Schicht gemessen wird, leidet die Spezifität des Tests, weil störende Probenbestandteile durch Waschen nicht entfernt werden können.

Die Möglichkeit, mehrere für eine immunologische Reaktion benötigte Reagenzien in einer Schicht eines Testelements auf engem Raum unterzubringen, wird in der älteren europäischen Patentanmeldung EP-A 0469445 beschrieben. Dabei nutzt man zur Herstellung der Reagenzienschicht ein Verfahren, welches ebenfalls in einer älteren europäischen Patentanmeldung, EP-A 0469444, beschrieben ist. Bei dem genannten Herstellungsverfahren wird in Ink-Jet-Technik, beispielsweise mit einem Mehrkanaldruckkopf, Reagenzflüssikeit auf ein Target, beispielsweise eine inerte Folie, aufgebracht. Dabei können präzise dosierte Flüssigkeitsquanten mit hoher Frequenz genau positioniert werden, so daß sich beliebige Muster, beispielweise abwechselnde, sich nicht berührende Streifen untereinander unverträglicher Reagenzien, erzeugen lassen. In EP-A 0469445 wird auf diese Weise ein Analysenelement ohne Chromatographieeigenschaften beschrieben, bei dem nebeneinander in enger räumlicher Nähe mehrere unterschiedliche Reagenzzonen vorliegen.

Aufgabe der Erfindung war es , die Nachteile der Analysenelemente des Standes der Technik zu beseitigen und ein Analysenelement zur Verfügung zu stellen, bei dem alle zur Testführung notwendigen Reagenzien auf einem Testelement integriert sind, und dennoch eine einfachere, schnellere und genauere immunologische Bestimmung von Analyten insbesondere von Proteinparametern ermöglicht wird.

Es wurde gefunden, daß diese Aufgabe mit einem Analysenelement gelöst wird, wie es in den Ansprüchen charakterisiert ist.

Gegenstand der Erfindung ist ein Analysenelement zur Bestimmung eines Analyten nach dem Prinzip eines heterogenen Immunoassays, bestehend aus einem porösen kapillaraktiven Trägermaterial mit einer Multifunktionszone, in der ein trägerfixierter spezifischer Bindungspartner immobilisiert ist, und einer die Multifunktionszone so umgebenden Chromatographiezone, daß gelöste Reagenzien durch Kapillarkräfte in mehr als einer Richtung aus der Multifunktionszone entfernt werden können, dadurch gekennzeichnet, daß in der Multifunktionszone alle für den heterogenen Immunoassay notwendigen Bindungspartner in der Weise aufgebracht sind, daß mindestens der analytspezifische und der markierte Bindungspartner in einer Anzahl eng benachbarter, aber räumlich voneinander getrennter Kompartimente vorliegen, wobei mindestens die Kompartimente des markierten Bindungspartners in feuchtem Zustand löslich sind.

In dem Testelement liegt das poröse, kapillaraktive Trägermaterial als flächenförmige Matrix vor. Die Fläche kann beispielsweise quadratisch, rechteckig oder kreisförmig ausgebildet sein; möglich ist aber auch ein entsprechendes Flächensegment, wie dies zum Beispiel ein Teststreifen darstellt. Als Material für die saugfähige Matrix kommen alle porösen Materialien in Frage, die aufgrund von Kapillarkräften Flüssigkeit und darin gelöste Reagenzien parallel zur Trägermaterialebene transportieren können und die nicht nachteilig mit den eingesetzten Reagenzien wechselwirken oder reagieren. Solche porösen Trägermaterialien sind dem Fachmann bekannt. Beispielsweise können Papier, Cellulose, Nitrocellulose, gepreßte Fasern, wie zum Beispiel Glasfasern, gesintertes Glas, Keramik oder Kunststoffe poröser oder faseriger Struktur mit hinreiched hydrophilen Eigenschaften eingesetzt werden. Besonders vorteilhaft kann Nitrocellulose eingesetzt werden. Die Wahl der Porengröße des Materials hängt von den jeweiligen Reaktionsbedingungen ab. Je größer die Poren, umso schneller der Flüssigkeitstransport, umso länger braucht andererseits die immunologische Reaktion. Als günstigste Porengröße haben sich die Bereiche 0,1 - 5 µm, bevorzugt 0,45 - 1 µm erwiesen. Die Matrixdicke liegt vorteilhafterweise zwischen 50 und 250 µm.

Auf dem porösen Trägermaterial befinden sich im wesentlichen zwei Funktionszonen. Bevorzugt in zentraler Position der Trägermaterialfläche befindet sich eine Multifunktionszone. Diese Zone wird so bezeichnet, weil auf ihr sowohl die Analyt-aufgabe, als auch die immunologische Reaktion, als auch die anschließende Detektion der gebundenen markierten Reaktionskomponenten erfolgt. Die Multifunktionszone stellt einen abgegrenzten Bereich dar, der dadurch charakterisiert ist, daß innerhalb dieses Bereichs sich alle für den heterogenen Immunoassay notwendigen Bindungspartner in einer Schicht befinden. Bevorzugt ist dieser Bereich kreisförmig, möglich sind aber auch andere Formate wie ein rechteckiges Format, zum Beispiel im Falle eines Teststreifens oder auch andere Formate. Die Flächengröße der Multifunktionszone richtet sich im wesentlichen nach der Menge der aufzubringenden Immunreagenzien. Bevorzugt ist die Multifunktionszone Teil des kapillaraktiven Trägermaterials selbst. Möglich ist aber auch, daß die Multifunktionszone von einer zusätzlichen kapillaraktiven Schicht gebildet wird, die auf das Trägermaterial aufgebracht ist und mit diesem in flüssigkeitstransportierendem Kontakt steht.

Die Multifunktionszone wird von einer Chromatographiezone zumindest teilweise umgeben. Diese hat die Aufgabe, überschüssige Flüssigkeit bzw. auf die Reaktionszone aufgegebene Waschflüssigkeit und überschüssige, frei bewegliche Reagenzien durch Kapillarkräfte in mindestens zwei verschiedenen Richtungen aus der Multifunktionszone zu entfernen und aufzunehmen. Im Falle einer kreisförmigen Multifunktionszone mit einer dieser vollständig umgebenden Chromatographiezone erfolgt der Flüssigkeitstransport aus der Multifunktionszone in die Chromatographiezone in alle Richtungen radial in der Trägermatrix nach außen. Im Falle eines Teststreifens erfolgt der Flüssigkeitstransport bedingt durch die Begrenzungen des Streifens im wesentlichen zu gleichen Teilen in beide Längsrichtungen des Streifens.

Zur Unterstützung der chromatographischen Wirkung des die Chromatographiezone bildenden Trägermaterials kann dieses in der Chromatographiezone mit einem flüssigkeitsabsorbierenden Material verbunden sein. Vorteilhaft ist, wenn die Absorptionskraft dieses Materials stärker ist als die Kapillarkraft des Trägermaterials selbst. Als geeignetes flüssigkeitsabsorbierendes Material hat sich beispielsweise Whatman 3 MM Papier erwiesen.

Die vorliegende Erfindung ist dadurch gekennzeichnet, daß auf der Multifunktionszone alle für den heterogenen Immunoassay notwendigen Bindungspartner aufgebracht sind. Die für den heterogenen Immunoassay der vorliegenden Erfindung notwendigen Bindungspartner setzen sich im wesentlichen aus folgenden Bindungspartnern zusammen:

Ein erster Bindungspartner ist trägerfixiert auf der Membran immobilisiert. Der erste Bindungspartner ist dabei nicht analytspezifisch, aber spezifisch für einen zweiten Bindungspartner (immobilisierter, nicht-analytspezifischer Bindungspartner). Ein zweiter Bindungspartner ist spezifisch an den ersten Bindungspartner bindefähig und ist andererseits analytspezifisch (analytspezifischer Bindungspartner). Dieser Bindungspartner kann in nicht-immobilisierter Form vorliegen, wird aber bei Kontakt mit dem ersten immobilisierten Bindungsparter an diesen gebunden. Erster und zweiter Bindungspartner bilden in diesem Falle zusammen betrachtet ein immobilisiertes analytspezifisches Bindungsreagenz. Soll der zweite, analytspezifische Bindungspartner von vornherein in der Multifunktionszone immobilisiert vorliegen, so kann dessen Trägerfixierung auch direkt, ohne über den Umweg der Bindung über einen ersten Bindungspartner erfolgen.
Ein dritter Bindungspartner ist die markierte Reaktionskomponente (markierter Bindungspartner). Dieser Bindungspartner darf nicht immobilisiert sein, daß heißt, er haftet zwar in trockenem Zustand des Analysenelementes in der Multifunktionszone, er muß aber bei Aufgabe der Analytlösung auf die Multifunktionszone frei in der Analytenflüssigkeit löslich sein.

Methoden der Markierung von Immunkomponenten und deren Detektion sind dem Fachmann vertraut. Bewährt haben sich Direktmarkierungen, die ohne weitere Reagenzien nachweisbar sind, wie Farbstoffmoleküle, Metallsole, Fluorophore, Luminophore oder z. B. Phycobiliproteine oder fluoreszierender Latex. Besonders vorteilhaft kann Fluoreszenzmarkierung eingesetzt werden.

Der dritte, markierte Bindungspartner hängt von dem immunologischen Testprinzip ab, das angewandt wird. Ist beispielsweise der Analyt ein Antigen oder ein Hapten, so kann der freie markierte Bindungspartner bei Verwendung eines kompetitiven Testprinzips ein dem Antigen analoges markiertes Antigen sein. Der zweite Bindungspartner ist in diesem Fall ein an den ersten Bindungspartner bindefähiger Antikörper. Markierter Bindungspartner und Antigen konkurrieren in diesem Falle um die Bindestellen des zweiten Bindungspartners, wobei die Menge des gebundenen markierten Analytanalogen ein Maß für die Konzentration des Analyten darstellt.

Bei Verwendung des Sandwich-Prinzips kann der freie, markierte Bindungspartner ein markierter analytspezifischer Antikörper sein. Zweiter Bindungspartner und markierter Bindungspartner binden das Analytantigen über verschiedene Epitope. Die Menge des gebundenen markierten Bindungspartners stellt ein Maß für die Konzentration des Analyten dar. Die Begriffe Antigen und Antikörper können in der oben gegebenen Beschreibung ausgetauscht werden.
Als erster Bindungspartner (immobilisiert und nicht-analytspezifisch) kann bevorzugt Streptavidin (oder Avidin) verwendet werden, das den zweiten, analytspezifischen Bindungspartner aufgrund dessen Konjugation mit einem Biotinmolekül spezifisch binden kann. Möglich sind aber auch alle dem Fachmann bekannten weiteren spezifischen Bindepaare wie Zucker/Lectin, komplementäre Nukleotidsequenzen, Enzym/Cofaktor, Antikörper/ Antigen usw.

Alle diese, für den heterogenen Immunoassay notwendigen Reaktionskomponenten (erster, zweiter und dritter Bindungspartner) sind erfindungsgemäß auf der Multifunktionszone gemeinsam aufgebracht. Dabei liegen zumindest die beiden Reaktionskomponenten analytspezifischer und markierter Bindungspartner auf einer Anzahl engbenachbarter aber doch räumlich voneinander getrennter sogenannter Kompartimente vor.

Der Begriff "Kompartiment" bezeichnet einen gegenüber den Dimensionen der Reaktionszone kleinen, flächenförmigen abgegrenzten Teilbereich der eine Reagenzspezies enthält. Ein Kompartiment kann aus einem Fleck, oder mehreren überlappenden Flecken oder aus einer Linie bestehen.

Die Kompartimente sind dadurch räumlich voneinander getrennt, da sie in sehr geringem Abstand nebeneinander in der Multifunktionszone angeordnet sind, wobei die Kompartimente mit verschiedenen Reagenzien bevorzugt alternieren, das heißt, daß wechselweise Kompartimente verschiedener Reagenzien benachbart sind. Aus praktischen Gründen ist ein regelmäßiges Alternieren zweckmäßig, bei dem sich also Kompartimente verschiedener Reagenzien in einer oder auch in zwei Flächenrichtungen zyklisch wiederholen, so daß sich ein regelmäßiges Linien- bzw. Punktmuster ergibt. In Ausnahmefällen kann aber auch ein alternierendes Muster ohne zyklische Wiederholung zweckmäßig sein. Idealerweise sollte der Abstand der Kompartimente unendlich klein sein. Auf der anderen Seite sollten sich die Kompartimente verschiedener Reagenzien in trockenem Zustand gerade nicht berühren.

Praktischerweise liegt der Abstand der äußeren Begrenzung verschiedener Kompartimente zwischen 10 µm und 1 mm, bevorzugt zwischen 30 µm und 250 µm, ganz besonders bevorzugt zwischen 40 µm und 100 µm. Die Breite der Kompartimentlinien bzw. der Durchmesser der Kompartimentflecken sollte vorteilhafterweise weniger als 2 mm betragen und liegt bevorzugt zwischen 50 µm und 1 mm. Diese Formatierung und Anordnung der Kompartimente wird im folgenden auch als Mikrokompartimentierung bezeichnet.

Je nach Testablauf, für den das erfindungsgemäße Analysenelement benutzt werden soll, können die Kompartimente sowohl eluierbare als auch an die Tragschicht immobilisierte Reagenzien enthalten, wobei in der Multifunktionszone ausschließlich Kompartimente mit eluierbaren Reagenzien ("eluierbare" oder "lösliche Kompartimente") oder eine Mischung aus Kompartimenten mit eluierbaren Reagenzien und immobilisierten Reagenzien ("immobilisierte Kompartimente") vorhanden sein können.

In einer Ausführungsform der Erfindung liegen nur die bei der Immunreaktion beteiligten Reaktionskomponenten - also zweiter und dritter Bindungspartner - auf Kompartimenten vor. Der erste, nicht-analytspezifische Bindungspartner wird großflächig auf der Multifunktionszone immobilisiert. Besonders bevorzugt wird dabei Streptavidin benutzt, das sich zum Beispiel direkt kovalent oder adsorptiv in Form von Polystreptavidin oder T-Rinderserumalbumin - Streptavidin immobilisieren läßt (siehe EP-A-0 344 578). Auf diese Schicht des ersten Bindungspartners in der Multifunktionszone werden die bei der immunologischen Nachweisreaktion beteiligten Reaktionskomponenten, also zweiter und dritter Bindungspartner, in Form von Kompartimenten aufgebracht. Durch Kontakt mit dem ersten Bindungspartner wird dabei auch der zweite Bindungspartner immobilisiert, während der dritte markierte Bindungspartner in eluierbaren Kompartimenten vorliegt. Insbesondere auf eine mit Streptavidin vorbehandelte Tragschicht können lösliche Kompartimente (mit nichtbiotinylierten Reagenzien) in einfacher Weise aufgebracht werden, ohne daß deren Löslichkeitsverhalten wesentlich beeinflußt wird. Nähere Einzelheiten sind der EP-A-0 344 578 zu entnehmen. Entscheidend ist, insbesondere bei einem kompetitiven Immunoassay, daß es durch die Kompartimentierung der Reagenzien zu keinem vorzeitigen Kontakt zwischen zweitem und drittem Bindungspartner vor der Analytzugabe kommt.

In einer anderen bevorzugten Ausführungsform der Erfindung werden alle Reaktionskomponenten in kompartimentierter Form in der Multifunktionszone aufgebracht. In diesem Falle befindet sich der erste Bindungspartner in immobilisierten Kompartimenten, während zweiter bzw. dritter Bindungspartner sich in löslichen Kompartimenten befinden. Da in diesem Falle die Membran in der Multifunktionszone nicht mehr vollflächig, sondern nur noch in Kompartimenten von dem ersten Bindungspartner bedeckt ist, empfiehlt es sich, als Trägermaterial solche Materialien auszuwählen, deren Oberfläche keine oder nur geringe unspezifische Wechselwirkungen mit dem Analyt oder den in löslichen Kompartimenten vorliegenden Immunreagenzien eingehen. Beispiele dafür sind modifizierte Nylonmembrane wie Loprodyne^{R} oder andere hydrophile Membranen wie hydrophiles Durapore^{R} der Firma Millipore.

Das Aufbringen der Immunreagenzien in Kompartimenten muß durch solche Verfahren erfolgen, die geeignet sind, mehrere verschiedene Immunreagenzien auf einer porösen Membranen in eng begrenzten und eng benachbarten Bereichen gezielt und in reproduzierbarer Weise aufzubringen, wobei verschiedene Reagenzien nebeneinander auf engstem Raum zum Teil eluierbar und zum Teil immobilisiert vorliegen. Das Aufbringen von Immunreagenzien in dieser Weise wurde bisher noch nicht beschrieben.

Als geeignet haben sich eine große Zahl verschiedener Drucktechniken herausgestellt. Dazu zählen Siebdrucktechnik, die aus der Computerdrucktechnik bekannten Techniken wie Ink-Jet in verschiedenen Variationen, Nadeldrucktechniken, Sprühtechniken wie Air-brush, "Charged drop"-Drucktechniken und andere.

Bei der Siebdrucktechnik wird durch ein feinmaschiges Raster zuerst eine Komponente auf das Trägermaterial aufgestrichen, dann das Raster verschoben und eine zweite und gegebenenfalls weitere Komponenten in die Lücken des ersten Rasters aufgestrichen. Dieses Verfahren eignet sich weniger, wenn mehr als zwei verschiedene Komponenten kompartimentiert werden sollen. Die Abstände der Kompartimente liegen eher an der oberen für die Erfindung noch tolerierbaren Grenze.

Eine Applikation von Immunreagenzien über Nadelapplikation eignet sich ebenfalls in solchen Fällen eher, indem die Abstände der Kompartimente nicht besonders klein zu sein brauchen.

Durch eine feine hohle Nadel von 0,05 - 1 mm Innendurchmesser wird mit einer exakt dosierenden Pumpe Reagenzflüssigkeit auf die Membran aufgebracht.

Die Strichstärke hängt davon ab, wie schnell die Nadel über die Membran bzw. die Membran unter der Nadel bewegt wird. Bevorzugt ist eine Applikation von Reagenzien mit nach oben stehender Nadelöffnung und darüber geführter Membran.

Bevorzugtere Verfahren im Sinne der Erfindung, sind Verfahren, die einen kleineren Abstand der Kompartimente erlauben. Dazu zählt beispielsweise die Applikation von Immunreagenzien mit dem sogenannten Airbrush-Verfahren. Dies ist ein kontinuierliches Sprühverfahren, bei dem, von einem Gasstrom umhüllt, Mikrotropfen auf eine Oberfläche bevorzugt in Linien aufgebracht werden. Der Gasstrom dient zum Ablenken und Positionieren des Mikrotropfenstrahls. Bekannt ist dieses Verfahren zum Aufbringen von Analytlösungen auf die Startlinie von Dünnschichtplatten.

Es hat sich herausgestellt, daß die hierfür kommerziell erhältlichen Geräte (zum Beispiel CAMAG DC-Probenautomat III) geeignet sind, verschiedene Immunreagenzien mikrokompartimentiert auf eine poröse Matrix aufzubringen. Dabei werden typischerweise Mikrotropfen der Größe 1 nl - 1000 nl, bevorzugt unter 100 nl, auf die Multifunktionszone des erfindungsgemäßen Analysenelementes in Punkt- oder Strichmustern, vorzugsweise in alternierender Folge miteinander unverträglicher Reagenzien aufgebracht. Die Dichte der Linien beziehungsweise Punkte ist dabei abhängig von der Saugfähigkeit des Trägermaterials und der Größe der Tropfen und liegt vorzugsweise zwischen 10 und 100 Linien pro cm² beziehungsweise 100 bis 10.000 Tropfen pro cm².

Im Sinne der Erfindung noch vorteilhafter sind die ursprünglich für Computerdrucker (Tintenstrahldrucker) entwickelten Ink-Jet-Techniken, weil sich mit diesen Verfahren noch kleinere Reagenzflüssigkeitsquanten schärfer abgetrennt aufbringen lassen. Unter den verschiedenen Methoden dieser Technologie ist die sogenannte Bubble-Jet-Technik ganz besonders bevorzugt. Ink-Jet-Techniken können in kontinuierliche und diskontinuierliche Techniken eingeteilt werden. Beide Gruppen sind für die Erfindung geeignet.

Die Anwendung zweier spezieller Varianten der diskontinuierlichen Ink-Jet-Technik zum Aufbringen eines Reagenzes auf einen räumlich abgegrenzten Bereich wird in EP-A-119 573 und EP-A-268 237 (US-A-4,877,745) beschrieben. Ein solcher abgegrenzter Bereich kann zum Beispiel die Form eines Plus- oder Minuszeichens haben, um das Analysenresultat deutlicher,beziehungsweise für den Laien verständlicher zu machen, oder er kann dazu dienen eine unmittelbaren Vergleich zwischen einem mit reagenzbeschichteten Teilbereich und einem reagenzfreien Teilbereich zu ermöglichen. In Bezug zur Ink-Jet-Technologie wird auf die oben genannten Schriften hier Bezug genommen. Die Ink-Jet-Technik zeichnet sich dadurch aus, daß sehr kleine Teilmengen einer Flüssigkeit in hoher Präzision auf eine Tragschicht appliziert werden können. Die Präzision bezieht sich dabei sowohl auf die exakte Positionierung des von dem Reagenztropfen erzeugten Punktes auf der Tragschicht, als auch auf das Reagenzvolumen. Die Tropfen können mit hoher Frequenz hintereinander ausgestoßen werden.

Es wurde gefunden, daß auch verschiedene Reagenzien auf kleinem Raum in eng benachbarten, aber räumlich voneinander getrennten Kompartimenten auf porösen Membranen mit verschiedenen Ink-Jet-Techniken appliziert werden können.

Zur Herstellung eines erfindungsgemäßen Analysenelementes liegt das Volumen eines von einem Ink-Jet-Düsenkopf ausgestoßenen Quantums Reagenzflüssigkeit typischerweise zwischen 2 und 2000 nl, bevorzugt zwischen 100 und 800 nl. Die Fläche des von einem solchen Quantums auf der Tragschicht erzeugten Punktes ist stark von der Reagenzflüssigkeit und der Tragschicht abhängig. Sie liegt etwa zwischen 3000 µm² und 0,1 mm² bevorzugt zwischen 500 µm² und 0,2 mm². Die Reagenzflüssigkeitsquanten werden typischerweise mit einer Frequenz von mehr als 1000 s⁻¹, bevorzugt zwischen 1000 und 200.000 s⁻¹ ausgestoßen. Nähere Einzelheiten können der am 06.02.92 veröffentlichten deutschen Patentanmeldung Aktenzeichen P 4024544.6 (EP-A 0469445) entnommen werden.

In den oben angegebenen Schriften EP-A-119573 und EP-A-268237 wurden zum flächenförmigen Applizieren von Reagenzien lediglich spezielle Varianten der Ink-Jet-Technologie benutzt, bei denen das Volumen einer Düsenkammer in einer Variante mechanisch, in der anderen Variante piezoelektrisch komprimiert wird, wenn ein Tropfen ausgestoßen werden soll.

Es hat sich herausgestellt, daß unter den Ink-Jet-Techniken die bisher noch nicht zum Applizieren von Reagenzien beschriebene Bubble-Jet-Technik auch vorteilhaft zur Herstellung des erfindungsgemäßen Analysenelementes benutzt werden kann.

Bei der ebenfalls von Computerdruckern bekannten Bubble-Jet-Technik wird ein Teilvolumen der Flüssigkeit in der Düsenkammer jeweils kurzfristig verdampft und expandiert, um ein Quantum der Flüssigkeit durch die Düse auszustoßen. Es sind dabei keine mechanisch beweglichen Teile mehr erforderlich, wodurch sich eine hohe Zuverlässigkeit ergibt. Ferner kann über einen breiten Viskositätsbereich der Flüssigkeit gearbeitet werden. Trotz der starken Erhitzung der Flüssigkeit in der Düsenkammer kommt es überraschenderweise zu keiner praktisch bedeutsamen Beschädigung von in der Flüssigkeit gelösten Immunreagenzien.

Zur Kompartimentierung der verschiedenen Reagenzien auf dem erfindungsgemäßen Analysenelement mit Ink-Jet-Techniken wird vorteilhafterweise mit einem Mehrkanaldruckkopf gearbeitet, wie sie für den Farbdruck entwickelt worden sind. Der Druckkopf kann mit einem von einer Steuereinheit gesteuerten X-Y-Antrieb in beiden Flächenrichtungen der Multifunktionszone positioniert werden. Ansonsten können die für die Ink-Jet-Technik, insbesondere die für die Bubble-Jet-Technik bekannten konstruktiven Einzelteile verwendet werden, die der Literatur über diese Techniken entnommen werden können. Nähere Einzelheiten können auch der am 06.02.92 veröffentlichten deutschen Patentanmeldung Aktenzeichen P 4024545.4 (EP-A 0469444) entnommen werden.

In einer besonderen Ausführungsform der Erfindung sind, innerhalb der Chromatographiezone, zusätzlich eine oder mehrere Kontrollzonen um die Reaktionszone angeordnet. Diese Kontrollzonen enthalten ein immobilisiertes spezifisches Abfangreagenz für den in der Reaktionszone löslich aufgebrachten markierten Bindungspartner. Dies kann z. B. ein gegen den freien markierten Bindungspartner gerichteter Antikörper sein. Mit Hilfe einer oder mehrerer solcher Kontrollzonen kann überprüft werden, ob der nach der Immunreaktion nicht-fixierte Teil der in der Reaktionszone aufgebrachten markierten Bindungspartner in die Chromatographiezone ausgewaschen wird und dort ein Signal erzeugt. So wird eine positive Reaktionskontrolle ermöglicht.

Gegenstand der Erfindung ist weiterhin ein Verfahren zur Bestimmung eines Analyten in einer Probenflüssigkeit nach dem Prinzip eines heterogenen Immunoassays unter Verwendung des erfindungsgemäßen Analysenelementes.

Das Verfahren ist dadurch gekennzeichnet, daß Analytlösung auf die Multifunktionszonen aufgegeben wird, mit einer Waschflüssigkeit die nicht in der Multifunktionszone immobilisierten Reagenzien in die Chromatographiezone gespült werden und in der Multifunktionszone die Menge des Analyten bestimmt wird.

Zur Durchführung einer Analytbestimmung auf dem erfindungsgemäßen Analysenelement wird Probenlösung, die den zu bestimmenden Analyten enthält, auf die Multifunktionszone aufgebracht. Bevorzugt erfolgt die Aufgabe auf die Mitte dieser Zone und das Volumen der Probenflüssigkeit sollte so groß sein, daß die gesamte Zone von Flüssigkeit benetzt wird. Nach beendeter Reaktion der Bindungspartner werden mit einer Waschflüssigkeit alle nicht auf der Multifunktionszone immobilisierten Reagenzien in zwei oder mehr Richtungen in die Chromatographiezone ausgewaschen. Als Waschflüssigkeit können für Immunreagenzien übliche Pufferlösungen benutzt werden, die nicht mit diesen nachteilig wechselwirken. Die Waschflüssigkeit wird vorteilhafterweise ein- oder mehrmals auf die Mitte der Multifunktionszone aufgebracht. Anschließend wird die Menge des in der Multifunktionszone oder in einem Teilbereich dieser Zone gebundenen markierten Bindungspartners als Maß für die Menge des Analyten gemessen. Die Messung kann auf der Membranseite erfolgen, auf der Analyt- und Waschlösung aufgegeben werden. Sie kann aber auch vorteilhafterweise auf der entgegengesetzten Seite des Trägermaterials durchgeführt werden.

Das erfindungsgemäße Analysenelement zeigt bei der Durchführung von heterogenen Immunoassays beträchtliche Vorteile. Durch die vollständige Integration aller Immunreagenzien auf einer Zone ist keine umständliche sukzessive Aufgabe von Reagenzien- und Waschlösungen mehr erforderlich. Der Test reduziert sich damit auf die Aufgabe der Analytlösung und anschließendes Waschen, wodurch die Zeitdauer des Tests und das Risiko von Fehlern erheblich reduziert wird.

Die Erfindung erlaubt den Einsatz genau abgemessener Reagenzien auf der Multifunktionszone und vermeidet die Notwendigkeit des Dosierens der Reagenzien durch den Benutzer. Die geringen Abstände der Reagenzkompartimente erlauben eine gute und schnelle Durchmischung der Reagenzien bei Flüssigkeitskontakt ohne zusätzliche Maßnahmen. Dies ermöglicht einen auch für ungeübte Benutzer sehr einfach und schnell durchführbaren Test, der innerhalb 10 - 300 Sekunden quantitative Testergebnisse liefert. Indem darüber hinaus völlig neue Testabläufe auf engstem Raum ermöglicht werden, werden besonders schnelle Testzeiten erreicht. Dies ist der Fall, wenn die Reaktion zwischen den eigentlichen Immunkomponenten (zweitem und drittem Bindungspartner) in homogener Phase abläuft und die Bindung an den Träger erst danach als heterogene Reaktion stattfindet. Dies wird dadurch ermöglicht, daß erfindungsgemäß Bindungspartner sowohl in immobilisierter als auch in löslichen Kompartimenten in der Multifunktionszone aufgebracht sind.

Es zeigt sich, daß die löslichen Bindungspartner von der Probe sehr schnell gelöst werden, so daß die Reaktion dieser Bindungspartner mit dem Analyten unmittelbar nach dem Probenkontakt erfolgt. Zugleich steht die gesamte Probe zu dem trägerfixierten Bindungspartner in Kontakt. Die Mikrokompartimentierung der Reagenzien ermöglicht einen schnellen und homogenen Ablauf der Analytbindungsreaktion mit einer sehr geringen Proben- und Reagenzmenge. Die Reaktion mit dem trägerfixierten Bindungspartner läuft als heterogene Reaktion langsamer ab und setzt praktisch erst nach Ablauf der homogenen Bindungsreaktion des Analyten ein.

Bei hoher Affinität von erstem und zweitem Bindungspartner läuft die heterogene Reaktion trotzdem noch in genügender Geschwindigkeit ab. Diesen Vorteil bietet z. B. das Streptavidin/Biotin-System.

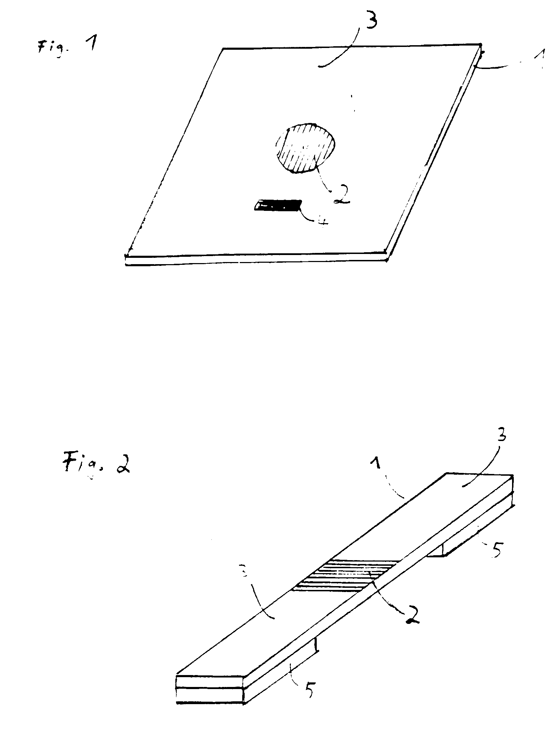

Im folgenden sind beispielhaft zwei spezielle Ausführungsformen eines erfindungsgemäßen Analysenelements beschrieben. Fig. 1 beschreibt ein näherungsweise quadratisches Analysenelement mit einer porösen Membran (1). Die Membran beinhaltet eine kreisförmige Multifunktionszone (2), die von einer Chromatographiezone (3) allseitig umgeben wird. Zusätzlich kann die Chromatographiezone eine oder mehrere Kontrollzonen (4) enthalten. Die Membran kann in einem Gehäuse eingebaut sein, das für die Aufgabe der Analytlösung und das Ablesen des Meßergebnisses der Multifunktionszone (2) und gegebenenfalls der Kontrollzone (4) über der Multifunktionszone (2) bzw. der Kontrollzone (4) eine Öffnung aufweist. Wird das Meßergebnis auf der unteren Membranseite abgelesen, enthält das Gehäuse unterhalb der Multifunktionszone eine weitere Öffnung.

Fig. 2 beschreibt ein Analysenelement in Form eines Teststreifens. Eine streifenförmige Membran (1) enthält in ihrer Mitte eine kreisförmige Multifunktionszone (2), deren Durchmesser der Streifenbreite entspricht, und eine sich in beide Richtungen der Membran anschließende Chromatographiezone (3). Die Membran kann mit einem saugfähigen Material (5) innerhalb der Chromatographiezone verbunden sein.

### Beispiel 1

### Kompetitiver Immunoassay auf T4

### 1. Herstellung einer Streptavidin-Matrix:

Nitrocellulose von Schleicher und Schüll AES 98 wurde mit einer Streptavidinlösung polymerisiert in PBS (1 mg/ml) getränkt und 30 Minuten bei 40° C getrocknet. Die Bindekapazität wurde durch Auftropfen von 10 µl einer 5 x 10⁻⁷ M Lösung von biotinyliertem B-Phycoerythrin (Sigma, München) geprüft. Nach zweimaligen Waschen konnte eine reproduzierbare Bindung von > 80 % durch Messung der fixierten Fluoreszenz festgestellt werden. Die Matrix wurde in 4 x 4 cm große Stücke zerschnitten.

### Applikation der Immunregenzien auf die Matrix:

Reagenz I: Biotinylierter T4 -Antikörper (5 x 10⁻⁷ M) in PBS-Puffer-Lösung mit 10 Gew.% Trehalose und 0,2 Gew.% 8-Anilino-1-Naphthalin-Sulfonsäure (ANS).

Reagenz II: T4-B-Phycoerythrin (10⁻⁶ M) in PBS-Puffer-Lösung mit 10 Gew.% Trehalose und 0,2 Gew.% ANS.

Reagenz I und Reagenz II wurden über Ink-Jet (Bubble-Jet) alternierend mit einem Hewlett Packard Paint-Jet-Mehrkanal-Druckkopf in Linien auf die Mitte der Membran aufgebracht. Die Liniendicke betrug ca. 250 µm, die Linien-abstände betrugen ebenfalls ca. 250 µm. Die Gesamtauftragsmenge an Reagenzien lag bei 0,5 µl/cm², die bedruckte Fläche (Multifunktionszone) hatte eine Längenausdehnung von ca. 1 cm in jede Flächenrichtung und stellte näherungsweise einen Kreis dar.

### Testdurchführung:

Die Membran wurde mit einem 4 x 4 cm großen Stück Whatman 3 MM Papier unterlegt, das in der Mitte ein kreisförmiges Loch von ca. 2 x 2 cm aufwies, und in eine Halterung eingebaut. 5 µl einer Analytprobe (Humanserum) aus einer Serumstandardreihe wurden auf die Mitte der Multifunktionszone mit einer Pipette aufgegegen. Nach 3 Minuten wurden 3 x 10 µl Waschpuffer (PBS + 0,1 Gew.% Tween 20) in 10 Sekunden Abständen auf die Mitte der Multifunktionszone aufgegeben und danach die Mitte der Zone an einem Hitachi 4010 Gerät bei Ex 515 nm, Em 580 nm vermessen.

| T4 Konzentration nmol/l | Fluoreszenzeinheiten |
|---|---|
| "0" | 32,5 |
| 47,6 | 26,5 |
| 94 | 17 |
| 175 | 13,5 |
| 328 | 9,5 |

Die Genauigkeit der Werte ist vergleichbar mit kommerziellen zeitaufwendigen Mehrschrittassays. Trotzdem kann eine präzise T4-Bestimmung in weniger als 6 Minuten erhalten werden.

## Patentansprüche

1. Analysenelement zur Bestimmung eines Analyten nach dem Prinzip eines heterogenen Immunoassays bestehend aus einem porösen kapillaraktiven Trägermaterial (1), mit einer Multifunktionszone (2), in der ein spezifischer Bindungspartner auf dem Trägermaterial (1) immobilisiert ist, und einer die Multifunktionszone (2) so umgebenden Chromatographiezone (3), daß gelöste Reagenzien durch Kapillarkräfte in mehr als einer Richtung aus der Multifunktionszone entfernt werden können,
dadurch gekennzeichnet, daß in der Multifunktionszone (2) alle für den heterogenen Immunoassay notwendigen Bindungspartner in der Weise aufgebracht sind, daß mindestens die analytspezifischen Bindungspartner und markierten Bindungspartner in einer Anzahl räumlich voneinander getrennter Kompartimente vorliegen, wobei die Kompartimente einen Abstand von weniger als 1 mm voneinander haben und mindestens die Kompartimente des markierten Bindungspartners in feuchtem Zustand löslich sind.

2. Analysenelement gemäß Anspruch 1, dadurch gekennzeichnet, daß der analytspezifische Bindungspartner in Kompartimenten immobilisiert und der markierte Bindungspartner in Kompartimenten in löslicher Form vorliegt.

3. Analysenelement gemäß Anspruch 1, dadurch gekennzeichnet, daß alle für den heterogenen Immunoassay notwendigen Bindungspartner in Kompartimenten vorliegen.

4. Analysenelement gemäß einem der Ansprüche 1 - 3, dadurch gekennzeichnet, daß der auf dem Trägermaterial (1) immobilisierte Bindungspartner Avidin oder Streptavidin ist.

5. Analysenelement gemäß einem der Ansprüche 1 - 4, dadurch gekennzeichnet, daß die analytspezifischen Bindungspartner und die markierten Bindungspartner aus biotinylierten Antikörpern und aus direkt markierten Antikörpern, die für einen Sandwich-Immunoassay geeignet sind, bestehen.

6. Analysenelement gemäß einem der Ansprüche 1 - 4, dadurch gekennzeichnet, daß die analytspezifischen Bindungspartner und die markierten Bindungspartner aus biotinylierten Antikörpern und direkt markierten Antigenen, die für einen kompetitiven Immunoassay geeignet sind, bestehen.

7. Analysenelement gemäß einem der Ansprüche 1 -6, dadurch gekennzeichnet, daß die Kompartimente eine Ausdehnung in eine Flächenrichtung von weniger als 2 mm haben.

8. Analysenelement gemäß einem der Ansprüche 1 - 7, dadurch gekennzeichnet, daß die Kompartimente Linien bilden.

9. Analysenelement gemäß einem der Ansprüche 1 - 8, dadurch gekennzeichnet, daß die Kompartimente Punkte bilden.

10. Analysenelement gemäß einem der Ansprüche 1 - 3 , dadurch gekennzeichnet, daß Kompartimente gleicher Reagenzien durch Kompartimente verschiedener Reagenzien voneinander getrennt sind.

11. Analysenelement gemäß einem der Ansprüche 1 - 10, dadurch gekennzeichnet, daß die Kompartimente über ein Ink-Jet-Verfahren aufgebracht sind.

12. Analysenelement gemäß Anspruch 11, dadurch gekennzeichnet, daß die Kompartimente über ein Bubble-Jet-Verfahren aufgebracht sind.

13. Analysenelement gemäß einem der Ansprüche 1 - 10, dadurch gekennzeichnet, daß die Kompartimente über Air-brush aufgebracht sind.

14. Analysenelement gemäß einem der Ansprüche 1 - 13, dadurch gekennzeichnet, daß die Chromatographiezone zusätzlich eine oder mehrere Kontrollzonen (4) enthält.

15. Verfahren zur Bestimmung eines Analyten nach dem Prinzip eines heterogenen Immunoassays, dadurch gekennzeichnet, daß die Analytlösung auf die Multifunktionszone (2) des Analysenelementes gemäß einem der Ansprüche 1 - 14 aufgebracht wird, daß mit einer Waschflüssigkeit die nicht auf der Multifunktionszone (2) immobilisierten Reagenzien in die Chromatographiezone (3) transportiert werden und in der Multifunktionszone (2) die Menge des Analyten bestimmt wird.

## Claims

1. Analytical element for the determination of an analyte according to the principle of a heterogeneous immunoassay comprising a porous capillary-active carrier material (1) with a multifunction zone (2) in which a specific binding partner is immobilized on the carrier material (1) and a chromatography zone (3) which surrounds the multifunction zone (2) in such a way that dissolved reagents can be removed by capillary forces from the multifunction zone in more than one direction, wherein all the binding partners necessary for the heterogeneous immunoassay are applied in the multifunction zone (2) in such a way that at least the analyte-specific binding partners and the labelled binding partners are present in a number of compartments that are spatially separated from one another, wherein the compartments are at a distance of less than 1 mm from one another and at least the compartments of the labelled binding partner are soluble in a wet state.

2. Analytical element as claimed in claim 1, wherein the analyte-specific binding partner is immobilized in compartments and the labelled binding partner is present in a soluble form in compartments.

3. Analytical element as claimed in claim 1, wherein all the binding partners necessary for the heterogeneous immunoassay are present in compartments.

4. Analytical element as claimed in one of the claims 1 - 3, wherein the binding partner immobilized on the carrier material (1) is avidin or streptavidin.

5. Analytical element as claimed in one of the claims 1 - 4, wherein the analyte-specific binding partners and the labelled binding partners are composed of biotinylated antibodies and of directly labelled antibodies which are suitable for a sandwich immunoassay.

6. Analytical element as claimed in one of the claims 1 - 4, wherein the analyte-specific binding partners and the labelled binding partners are composed of biotinylated antibodies and directly labelled antigens which are suitable for a competitive immunoassay.

7. Analytical element as claimed in one of the claims 1 - 6, wherein the dimension of the compartments in a planar direction is less than 2 mm.

8. Analytical element as claimed in one of the claims 1 - 7, wherein the compartments form lines.

9. Analytical element as claimed in one of the claims 1 - 8, wherein the compartments form points.

10. Analytical element as claimed in one of the claims 1 - 9, wherein compartments of the same reagents are separated from compartments of different reagents.

11. Analytical element as claimed in one of the claims 1 - 10, wherein the compartments are applied by means of an ink-jet process.

12. Analytical element as claimed in claim 11, wherein the compartments are applied by means of a bubble-jet process.

13. Analytical element as claimed in one of the claims 1 - 10, wherein the compartments are applied by an air-brush.

14. Analytical element as claimed in one of the claims 1 - 13, wherein the chromatography zone additionally contains one or several control zones (4).

15. Method for the determination of an analyte according to the principle of a heterogeneous immunoassay, wherein the analyte solution is applied to the multifunction zone (2) of the analytical element as claimed in one of the claims 1 - 14, the reagents that are not immobilized on the multifunction zone (2) are transported with a wash liquid into the chromatography zone (3) and the amount of the analyte is determined in the multifunction zone (2).

## Revendications

1. Elément d'analyse pour déterminer un analyte selon le principe d'un essai immunologique hétérogène constitué d'une matière support (1) poreuse à capillarité active, avec une zone multifonctionnelle (2), dans laquelle un partenaire de liaison specifique est immobilisé sur la matière support (1), et d'une zone de chromatographie (3) entourant la zone multifonctionnelle (2) de sorte que des réactifs dissous puissent être entraînés hors de la zone multifonctionnelle par des forces de capillarité dans plus d'une direction,
caractérisé en ce que dans la zone multifonctionnelle (2), tous les partenaires de liaison nécessaires à l'essai immunologique hétérogène sont déposés de sorte qu'au moins les partenaires de liaison spécifiques à l'analyte et des partenaires de liaison marqués se trouvent dans un certain nombre de compartiments spatialement séparés les uns des autres, les compartiments étant séparés par une distance inférieure à lmm et les contenus d'au moins les compartiments du partenaire de liaison marqué étant solubles à l'état mouillé.

2. Elément d'analyse selon la revendication 1, caractérisé en ce que le partenaire de liaison spécifique à l'analyte se trouve immobilisé dans des compartiments et le partenaire de liaison marqué se trouve dans des compartiments sous une forme.

3. Elément d'analyse selon la revendication 1, caractérisé en ce que tous les partenaires de liaison nécessaires à l'essai immunologique hétérogène se trouvent dans des compartiments.

4. Elément d'analyse selon l'une quelconque des revendications 1 à 3, caractérisé en ce que le partenaire de liaison immobilisé sur la matière support (1) est l'avidine ou la streptavidine.

5. Elément d'analyse selon l'une quelconque des revendications 1 à 4, caractérisé en ce que les partenaires de liaison spécifiques à l'analyte et les partenaires de liaison marqués sont constitués d'anticorps biotinylés et d'anticorps directement marqués qui conviennent à un essai immunologique en sandwich.

6. Elément d'analyse selon l'une quelconque des revendications 1 à 4, caractérisé en ce que les partenaires de liaison spécifiques à l'analyte et les partenaires de liaison marqués sont constitués d'anticorps biotinylés et d'anticorps directement marqués qui conviennent à un essai immunologique compétitif.

7. Elément d'analyse selon l'une quelconque des revendications 1 à 6, caractérisé en ce que les compartiments ont une étendue dans une direction de surface inférieure à 2 mm.

8. Elément d'analyse selon l'une quelconque des revendications 1 à 7, caractérisé en ce que les compartiments forment des lignes.

9. Elément d'analyse selon l'une quelconque des revendications 1 à 8, caractérisé en ce que les compartiments forment des points.

10. Elément d'analyse selon l'une quelconque des revendications 1 à 9, caractérisé en ce que les compartiments de réactifs identiques sont séparés, les uns des autres, par des compartiments de réactifs divers.

11. Elément d'analyse selon l'une quelconque des revendications 1 à 10, caractérisé en ce que les compartiments sont déposés au moyen d'un procédé à jet d'encre.

12. Elément d'analyse selon la revendication 11, caractérisé en ce queles compartiments sont déposés au moyen d'un procédé à bulle d'encre.

13. Elément d'analyse selon l'une quelconque des revendications 1 à 10, caractérisé en ce que les compartiments sont déposés au moyen d'un pinceau à air.

14. Elément d'analyse selon l'une quelconque des revendications 1 à 13, caractérisé en ce que la zone de chromatographie contient une ou plusieurs zones de contrôle (4).

15. Procédé pour déterminer un analyte selon le principe d'un essai immunologique hétérogène, caractérisé en ce que la solution d'analyte est déposée sur la zone multifonctionnelle (2) de l'élément d'analyse selon l'une quelconque des revendications 1 à 14, en ce que les réactifs non immobilisés sur la zone multifonctionnelle (2) sont transportés dans la zone de chromatographie (3) avec un liquide de lavage et que la quantité de l'analyte est déterminée dans la zone multifonctionnelle (2).
